# EUROPEAN PATENT APPLICATION

(11) **EP 4 591 877 A1**
(43) Date of publication of application: **30.07.2025**
(21) Application number: 24160717.5
(22) Date of filing: 29.02.2024
(51) Int. Cl.: A61K 39/00, A61K 35/17, C12N 5/10, A61P 35/02

(54) **NOVEL MARROW-INFILTRATING LYMPHOCYTES POPULATION COMPRISING CHIMERIC ANTIGEN RECEPTOR SPECIFIC FOR BCMA AND PRODUCING METHOD FOR THE SAME**

(30) Priority: 24.01.2024 KR 20240011223
(71) Applicant: Vaxcell-Bio, Jeollanam-do 58141 (KR); Industry Foundation of Chonnam National University, Buk-gu Gwangju (KR); Hebei Senlang Biotechnology Co. Ltd., Shijiazhuang High-tech Zone Hebei (CN)
(72) Inventor: Je Jung, Lee, Gwangju (KR); Vo Manh, Cuong, Jeollanam-do (KR); Jung Sung, Hoon, Seoul (KR); Wang, Lin, Hebei (CN)
(74) Representative: WP Thompson

(57) **Abstract**

The present invention relates to a novel population of marrow infiltrating lymphocytes (MILs) comprising a chimeric antigen receptor comprising a binding domain that is specific for BCMA; and a method for producing the same. Specifically, the present invention provides a novel population of MILs that is characterized by exhibiting target cell specificity and strong cytotoxicity against target cells due to BCMA CAR, and a method of culturing MILs capable of providing the same, and the novel population of MILs produced by the above method can be used as a new anticancer immunotherapy due to its high anticancer effect.

## Description

### Technical Field

The present invention relates to a novel population of marrow infiltrating lymphocytes (MILs) comprising a chimeric antigen receptor that is specific for BCMA (B cell maturation antigen); and a method for producing the same. Specifically, the present invention provides a novel population of MILs comprising a chimeric antigen receptor that is specific for BCMA, the population of MILs specifically binding to a target cell expressing BCMA and having strong cytotoxicity, and a method of culturing MILs capable of producing the same, and the population of MILs can be used as a new anticancer immunotherapy due to its high anticancer effect.

### Background Art

Multiple myeloma (MM) is a disease that is still difficult to treat effectively despite the development of several effective treatment methods, including proteasome inhibitors, immunomodulators, or monoclonal antibodies. Patients who are completely cured of multiple myeloma are very rare, and most patients with multiple myeloma are refractory to treatment or have multiple myeloma recurrence. Therefore, there is a need to develop a novel method for treating multiple myeloma that can overcome the above problems.

Adoptive T cell therapy, also known as cellular immunotherapy, has made significant progress in the treatment of multiple myeloma over the past decade with several licensed chimeric antigen receptor (CAR)-T cell therapies. However, current adoptive immunotherapies use peripheral blood lymphocytes (PBLs) as a source of T cells, and the PBLs lack intrinsic tumor specificity and are susceptible to tumor evasion and loss of antigen, which is one of the major drawbacks of CAR-T cells generated by PBLs. One way to overcome these drawbacks and increase the tumor specificity of adoptive T cell therapy is to use tumor infiltrating lymphocytes (TILs) comprising polyclonal memory T cells that target several tumor-related antigens obtained from solid tumors of patients with cancer. However, TILs cannot be applied to all patients with solid tumor. For example, patients with so-called cold tumors with low immunogenicity do not have TILs, and TILs therapy for solid tumors is not practical for a variety of reasons, including the long expansion time and high cost due to the need for high concentrations of IL-2.

### Prior Art Document

### Non-Patent Document

(Non-Patent Document 1) Badalamenti G, Fanale D, Incorvaia L, Barraco N, Listµ A, Maragliano R, et al. Role of tumor infiltrating lymphocytes in patients with solid tumors: Can a drop dig a stone? Cell Immunol. 2019;343:103753.
(Non-Patent Document 2) Kimberly AN IM. Marrow Infiltrating Lymphocytes: Their Role in Adoptive Immunotherapy. The Cancer Journal. 2015;21(6):501-5. doi: 10.1097/PPO.0000000000000159.
(Non-Patent Document 3) Sponaas AM YR, Rustad EH, Standal T, Thoresen AS, Vo CD, et al. PD1 is expressed on exhausted T cells as well as virus specific memory CD8+ T cells in the bone marrow of myeloma patients. Oncotarget. 2018;9(62):32024-32035. doi: 10.18632/oncotarget.25882.

### Disclosure of the Invention

### Technical Problem

BCMA is an abbreviation for B cell maturation antigen, and is also referred to as CD269, TNFRSF17, and the like. They are selectively expressed in B cells and are involved in cell survival and proliferation. BCMA has been shown to be expressed in several cancers comprising B cells, such as malignant B cells, multiple myeloma, and Hodgkin's lymphoma.

An object of the present invention is to provide a method for treating carcinomas with BCMA expression, such as multiple myeloma, by targeting BCMA.

The inventors of the present invention have generated MIL, an ex vivo expanded and activated MIL (eMIL) isolated from a patient, expressing a chimeric antigen receptor that specifically binds to BCMA, and have evaluated the immunological properties and cytotoxicity of these CAR-expressing eMILs and have demonstrated its superiority. Based on the above, the present inventors have completed the present invention.

Another object of the present invention is to provide a population of MILs expressing an antigen binding site, preferably a chimeric antigen receptor, that specifically binds to a cancer antigen, preferably BCMA, a method for producing the same, and a therapeutic use thereof. The therapeutic use of the present invention is preferably for the treatment of multiple myeloma.

### Effects of Invention

The marrow infiltrating lymphocytes comprising a chimeric antigen receptor that is specific for BCMA according to the present invention have excellent targeting efficiency by comprising two nanobodies specific for different epitopes of BCMA, and exhibit excellent anticancer effects against cancer, especially multiple myeloma, by effectively inducing marrow infiltrating lymphocytes into target cells expressing BCMA. In addition, the marrow infiltrating lymphocytes comprising a chimeric antigen receptor that is specific for BCMA according to the present invention exhibit a high proportion of CD8⁺ T_{cm} (central memory T cells), and this high proportion of CD8⁺ T_{cm} can help the marrow infiltrating lymphocytes have long-lasting cytotoxicity in the body. The BCMA CAR-MILs comprising a chimeric antigen receptor according to the present invention exhibit an increased cytotoxicity, such as an increased proportion of CD107a, compared to MILs that do not comprise a chimeric antigen receptor, and can therefore be effectively used in the prevention or treatment of cancer. In addition, the CAR-MILs according to the present invention have an excellent immuno-anticancer ability, for example, cytotoxic ability against cancer cells, than other types of T cells comprising the same CAR.

### Brief Description of Drawings

Fig. 1 is a schematic diagram showing the composition of CAR and the method for producing the population of CAR-MILs according to the present invention.
Fig. 2 is a graph obtained by flow cytometry showing the CAR expression rate of the population of CAR-MILs according to the present invention over time after transduction of the CAR according to the present invention into MILs.
Fig. 3 is a line graph showing the CAR expression rate of the population of CAR-MILs according to the present invention over time after transduction of the CAR according to the present invention into MILs.
Fig. 4 illustrates the results of flow cytometry showing the change in the proportion of CD3⁺ CD56⁻ T cells and cytokine-induced killer cells (CD3⁺ CD56⁺ T cells) in the population of CAR-MILs over time after transduction of the CAR according to the present invention into MILs.
Fig. 5 is a bar graph showing the change in CD3⁺ CD56⁻ T cells and cytokine-induced killer cells (CD3⁺ CD56⁺ T cells) in the population of CAR-MILs over time after transduction of the CAR according to the present invention into MILs.
Fig. 6 is a graph showing the expansion by proliferation of the population of CAR-MILs over time after transduction of the CAR according to the present invention into MILs.
Fig. 7 illustrates the results of flow cytometry showing the change in the proportion of CD8⁺ and CD4⁺ T cells in the population of CAR-MILs over time after transduction of the CAR according to the present invention into MILs.
Fig. 8 is a bar graph showing the change in CD8⁺ and CD4⁺ T cells in the population of CAR-MILs over time after transduction of the CAR according to the present invention into MILs.
Fig. 9 is a graph showing the results of flow cytometry showing the change in the proportion of naive T cells (CD62L⁺ CD45RA⁺ cells), central memory T cells (CD62L⁺ CD45RA⁻ cells), Tₑₘᵣₐ cells (CD62L⁻ CD45RA⁺ cells), and effector memory T cells (CD62L⁻ CD45RA⁻ cells) in the population of CAR-MILs over time after transduction of the CAR according to the present invention into MILs.
Fig. 10 is a bar graph showing the change in the proportion of naive T cells (CD62L⁺ CD45RA⁺ cells), central memory T cells (CD62L⁺ CD45RA⁻ cells), Tₑₘᵣₐ cells (CD62L⁻ CD45RA⁺ cells), and effector memory T cells (CD62L⁻ CD45RA⁻ cells) in the population of CAR-MILs over time after transduction of the CAR according to the present invention into MILs.
Fig. 11 is a graph showing the results of flow cytometry showing the change in the expression rate of immune checkpoint molecules on the surface of the population of CAR-MILs over time after transduction of the CAR according to the present invention into MILs.
Fig. 12 is a bar graph showing the change in the expression rate of immune checkpoint molecules on the surface of the population of CAR-MILs over time after transduction of the CAR according to the present invention into MILs.
Fig. 13 is a graph showing the results of flow cytometry showing the change in the proportion of CD4⁺CD25⁺Foxp3⁺ regulatory T cells (T_{reg}), CD8⁺CD25⁺Foxp3⁺ regulatory T cells (T_{reg}), and CD4⁺CD8⁺CD25⁺Foxp3⁺ regulatory T cells (T_{reg}) in the population of CAR-MILs over time after transduction of the CAR according to the present invention into MILs.
Fig. 14 is a bar graph showing the change in the proportion of CD4⁺CD25⁺Foxp3⁺ regulatory T cells (T_{reg}), CD8⁺CD25⁺Foxp3⁺ regulatory T cells (T_{reg}), and CD4⁺CD8⁺CD25⁺Foxp3⁺ regulatory T cells (T_{reg}) in the population of CAR-MILs over time after transduction of the CAR according to the present invention into MILs.
Fig. 15 is a graph showing the expression rate of CD107a on CAR-MILs when the population of CAR-MILs was co-cultured with cancer cell lines or cancer cells isolated from patients on days 9, 14, and 19 after transduction of the CAR according to the present invention into MILs.
Fig. 16 is a graph showing the cell death rate of cancer cells when the population of CAR-MILs was co-cultured with cancer cell lines or cancer cells isolated from patients on day 14 and day 19 after transduction of the CAR according to the present invention into MILs.
Fig. 17 is a graph showing that the number of cancer cells is reduced as target cancer cells are killed by the population of CAR-MILs over time when the population of CAR-MILs cultured in vitro is co-cultured with autologous cancer cells isolated from a patient or the RPMI8226 cancer cell line for 14 days after transduction of the CAR according to the present invention into MILs.

### Detailed Description for Carrying out the Invention

Hereinafter, with reference to the accompanying drawings, the present invention and embodiments will be described in detail so that those of ordinary skill in the art to which the present invention belongs can easily practice the present invention. However, the present invention can be implemented in various forms and is not limited to the embodiments described herein.

In the present invention, immune cells may refer to cells of hematopoietic origin that are functionally involved in the initiation and/or execution of innate and/or acquired immune responses. In the present invention, immune cells may be derived from stem cells. The stem cells may be adult stem cells, non-human embryonic stem cells, non-human stem cells, cord blood stem cells, progenitor cells, bone marrow stem cells, induced pluripotent stem cells, pluripotent stem cells, or hematopoietic stem cells.

The immune cells may be white blood cells, neutrophils, eosinophils, basophils, monocytes, lymphocytes, T cells, cytotoxic T cells, natural killer T cells, dendritic cells, or a combination thereof, but are not limited thereto.

As used herein, the term "marrow infiltrating lymphocyte (referred to as "MILs" or "MII,")" refers to a lymphocyte derived from the bone marrow. As used herein, the term "eMILs" refers to MILs activated by contact and culture with anti-CD3 and anti-CD28 antibodies under interleukin. As used herein, the term "MILs" may refer to eMII,s depending on the context.

As used herein, the term "activation" in relation to lymphocytes refers to the initiation of proliferation and differentiation of lymphocytes by mediators such as antigen specific receptors or interleukins, and an increase in anticancer function.

As used herein, the term "expansion" refers to an increase in the number of cells by proliferation.

Marrow infiltrating lymphocytes have other distinguishable differences from tumor infiltrating lymphocytes (TIL) as well as peripheral blood lymphocytes (referred to as "PBL" or "PBLs"). The bone marrow microenvironment represents a special immunological niche due to its abundance of antigen-presenting cells. The presence of these antigen-presenting cells maintains higher levels of central memory cells, such as those found in the bone marrow compartment, where processing and presentation of antigens occurs (Li JM et al J Immunol. 2009 Dec 15;183(12):7799-809). These MILs express markers of memory T cells, such as CD45RO⁺ and CD62L⁺, and have more memory cells than memory cells found in the population of PBLs (Noonan K et al Clin Cancer Res. 2012 Mar 1;18(5):1426-34). In addition, MILs exhibit different properties than tumor infiltrating lymphocytes (TILs) of hematological malignancies due to their ability to continuously prime memory cells with antigens (Beckhove P et al J Clin Invest. 2004 Jul 1; 114 (1):67-76, Castiglioni P et al 6 J Immunol 2008; 180:4956-4964). For example, MIL, unlike TIL, can be harvested from all patients and expanded in vitro (Noonan, K et al. Sci. Transl Med. 2015 May 20; 7(288): 288ra78). TILs are found in only about 50% of patients, and only about 25% of patients comprise expandable TILs. In addition, in contrast to PBLs, MILs possess a broad endogenous antigen repertoire that accounts for their intrinsic tumor specificity that is not shown in PBLs (Noonan et al Clin Cancer Res).

The source of MILs for the population of CAR-MILs according to the present invention can be obtained from patients with any of a number of types of cancer, including hematological malignancies and solid tumors, preferably patients with multiple myeloma. Preferably, it can be obtained from bone marrow cells, which have increased tumor specificity compared to peripheral blood.

In the present invention, "chimeric antigen receptor (CAR)" refers to a protein comprising an intracellular signaling domain and an extracellular binding domain. A chimeric antigen receptor (CAR) is composed of a binding domain capable of recognizing antigens, a signal sequence, a hinge region, a transmembrane domain, and an intracellular signaling domain, and they target a tumor-related antigen that is specific for a binding domain, thereby enabling immune cells to specifically attack cancer cells.

The inventors of the present invention prepared a CAR comprising a BCMA dual epitope binding nanobody and a population of CAR-MILs comprising the CAR using bone marrow-derived mononuclear cells (BMMNC) from patients with multiple myeloma, which showed a high delivery efficiency and a strong anti-myeloma immune response. In addition, the inventors of the present invention demonstrated that the population of CAR-MILs is an innovative immunotherapy approach for multiple myeloma. Based on the above, the present invention was completed.

In the present invention, "BCMA (B cell maturation antigen; UniProt ID: Q02223)" is an abbreviation for B cell maturation antigen, and is also referred to as CD269, TNFRSF17, and the like. They are selectively expressed in B cells and are involved in cell survival and proliferation.

### Chimeric antigen receptor according to the present invention

The binding domain included in the chimeric antigen receptor of the present invention specifically recognizes BCMA, and the chimeric antigen receptor of the present invention may have at least 70%, preferably at least 80%, and more preferably at least 90%, 95%, 97%, 99% or 100% sequence identity with the amino acid sequence of SEQ ID NO: 1.

The chimeric antigen receptor of the present invention specifically recognizes BCMA and is therefore also referred to as BCMA CAR.

In the present invention, "nanobody" refers to a single-domain antibody (sdAb), and has high stability and tissue penetration due to the small molecular weight resulting from a single domain.

Nanobodies are the smallest stable antibody molecules known to date and have high affinity for antigens, and due to their small size, they powerfully penetrate not only tumor tissue but also the blood-brain barrier, while exhibiting low toxicity and immunogenicity.

The binding domain included in the chimeric antigen receptor of the present invention includes VHH01 and VHH02, which are nanobodies specific for BCMA, and the above nanobodies may have at least 70%, preferably at least 80%, and more preferably at least 90%, 95%, 97%, 99% or 100% sequence identity with the amino acid sequences of SEQ ID NOs: 8 and 9, respectively.

The binding domain included in the chimeric antigen receptor of the present invention may include a VHH01 nanobody comprising the amino acid sequence of SEQ ID NO: 8 and a VHH02 nanobody comprising the amino acid sequence of SEQ ID NO: 9.

The nanobodies VHH01 and VHH02, which enable dual epitope binding to BCMA, can induce BCMA CAR-expressing T cells even with cancer cells expressing low levels of target antigen due to their high specificity when including VHH01 and VHH02 in the CAR. The dual nanobody CAR can show greater activity than a single nanobody CAR and can efficiently eliminate target tumor cells.

The VHH01 and VHH02 may comprise CDRs having at least 70%, preferably at least 80%, and more preferably at least 90%, 95%, 97%, 99% or 100% sequence identity with the amino acid sequence of SEQ ID NOs: 2 to 7.

The VHH01 and VHH02 may comprise CDR1 of SEQ ID NO: 2 or 3, CDR2 of SEQ ID NO: 4 or 5, and CDR3 of SEQ ID NO: 6 or 7.

The VHH01 may comprise CDR1 of SEQ ID NO: 2, CDR2 of SEQ ID NO: 4, and CDR3 of SEQ ID NO: 6, and the VHH02 may comprise CDR1 of SEQ ID NO: 3, CDR2 of SEQ ID NO: 5, and CDR3 of SEQ ID NO: 7.

The chimeric antigen receptor of the present invention may further comprise one or more selected from the group consisting of a binding domain that specifically binds to BCMA, a signal sequence, a hinge and transmembrane domain (TM), and an intracellular domain (intracellular signaling domain)

The hinge and transmembrane domain included in the chimeric antigen receptor of the present invention is preferably a hinge and transmembrane domain derived from CD8α, but is not limited thereto, and may preferably comprise the amino acid sequence of SEQ ID NO: 10, and may have at least 70%, preferably at least 80%, and more preferably at least 90%, 95%, 97% or 99% sequence identity with the amino acid sequence of SEQ ID NO: 10.

The intracellular signaling domain according to the present invention is a part located inside the cell membrane of an immune cell, that is, in the cytoplasm, and refers to a site that activates the immune response of an immune cell when an antibody bound to the extracellular domain binds to a target antigen.

The intracellular domain of the chimeric antigen receptor of the present invention may include two or more intracellular signaling domains. The intracellular signaling domain is preferably an intracellular signaling domain derived from 4-1BB connected to an intracellular signaling domain derived from CD3ζ, but is not limited thereto. Preferably, the intracellular signaling domain derived from 4-1BB may comprise the amino acid sequence of SEQ ID NO: 11, and the intracellular signaling domain derived from CD3ζ may comprise the amino acid sequence of SEQ ID NO: 12, and may have at least 70%, preferably at least 80%, and more preferably at least 90%, 95%, 97% or 99% sequence identity with the amino acid sequence of SEQ ID NO: 11 or SEQ ID NO: 12.

The population of CAR-MILs according to the present invention may be a population that exhibits an increased proportion of CD8⁺ T cells and a reduced proportion of CD4⁺ T cells compared to the MIL population that is not activated in vitro and does not comprise a chimeric antigen receptor that specifically binds to B cell maturation antigen (in other words, the MIL population just isolated from a patient).

As used herein, "just isolated" means that the MIL population isolated from the patient has not been expanded and activated in vitro.

The CAR-MILs according to the present invention may be a population that exhibits a reduced proportion of regulatory T cells compared to the MIL population that is not activated in vitro and does not comprise a chimeric antigen receptor that specifically binds to B cell maturation antigen (in other words, the MIL population just isolated from a patient).

The CAR-MILs according to the present invention may be a population that exhibits an increased proportion of CD62L⁺ CD45RA⁻ central memory T cells compared to the MIL population that is not activated in vitro and does not comprise a chimeric antigen receptor that specifically binds to B cell maturation antigen (in other words, the MIL population just isolated from a patient).

The CAR-MILs according to the present invention may be a population that exhibits a reduced expression level of PD-1 (programmed cell death protein 1) compared to the MIL population that is not activated in vitro and does not comprise a chimeric antigen receptor that specifically binds to B cell maturation antigen (in other words, the MIL population just isolated from a patient).

The CAR-MILs according to the present invention may exhibit an increased proportion of CD8⁺ T cells and central memory T cells, a reduced proportion of CD4⁺ T cells and regulatory T cells, and a low expression level of PD-1 compared to the MIL population that is not activated in vitro and does not comprise a chimeric antigen receptor that specifically binds to B cell maturation antigen (in other words, the MIL population just isolated from a patient).

The CAR-MILs according to the present invention may exhibit excellent cytotoxic capacity against CD138⁺ primary multiple myeloma cells with a higher expression rate of CD107a compared to the isolated population of PBLs or MILs that is not activated in vitro and does not comprise a chimeric antigen receptor that specifically binds to B cell maturation antigen. In addition, the CAR-MILs according to the present invention have excellent immuno-anticancer abilities, such as cytotoxicity against cancer cells, than other types of T cells comprising the same CAR.

The proportion of different types of immune cells that make up an immune cell population such as MILs can be determined by conventional experimental techniques, such as flow cytometry. Different types of immune cells can be distinguished by the presence of protein(s) that is each uniquely expressed on the cell surface.

As used herein, the term "differentiation" refers to the developmental process by which cells acquire, for example, morphological characteristics and/or functions that allow them to become specialized for a particular function.

As used herein, the term "substantially fully differentiated" means that a cell is mature and is finally differentiated into a fully differentiated cell, and means that 90% or more, 95% or more, or 100% of the cells are differentiated.

As used herein, the term "memory T cell" is a type of T cell and refers to an antigen-specific T cell that remains for a long time even after the antigen has been removed. For example, it is characterized by the presence of CD4 or CD8 protein on the cell surface. This refers to a cell that quickly converts to an effector T cell and responds to the antigen when re-exposed to a specific antigen.

As used herein, the term "regulatory T cell" is a type of T cell and refers to a T cell that performs the function of maintaining homeostasis and self-tolerance by suppressing immune responses. For example, it is characterized by the presence of one or more proteins selected from CD4 or CD8 and CD25 and Foxp3 proteins on the cell surface, and is also referred to as Treg.

As used herein, the term "central memory T cell" refers to a T cell that has a high self-renewal ability and is mainly present in the lymph nodes and peripheral circulatory system. For example, it is characterized by the presence of the CD62L protein on the cell surface and the absence of the CD45RA protein on the cell surface, and is also referred to as T_{cm}.

As used herein, the term "CD4⁺" means that CD4 (cluster of differentiation 4), a glycoprotein that acts as a co-receptor for the T cell receptor (TCR), is present on the cell surface.

As used herein, the term "CD8⁺" means that CD8 (cluster of differentiation 8), a glycoprotein that acts as a co-receptor for the T cell receptor (TCR), is present on the cell surface.

As used herein, the term "CD138⁺" means that the CD138 protein, also referred to as syndecan-1, is present on the cell surface. CD138 is a member of the syndecan proteoglycan family, functions as a receptor for an extracellular matrix, is involved in cell differentiation, and exhibits increased expression in various types of cancer.

As used herein, the term "CD107a" refers to the CD107a protein, and is lysosome-associated membrane glycoprotein 1, also referred to as LAMP-1, a protein involved in the degranulation of immune cells.

As used herein, the superscript "+" indicates the presence of the indicated protein on the cell surface.

As used herein, the subscript "-" indicates the absence of the indicated protein on the cell surface.

### Method for producing eMIL comprising BCMA CAR according to the present invention

The present invention provides a method for producing an isolated population of marrow infiltrating lymphocytes comprising a BCMA CAR, and a population of marrow infiltrating lymphocytes produced by the above method.

The method comprises the steps of: isolating marrow infiltrating lymphocytes from the bone marrow of a patient with cancer; culturing the isolated marrow infiltrating lymphocytes by contacting them with anti-CD3 and anti-CD28 antibodies in the presence of IL-2; transducing the isolated marrow infiltrating lymphocytes with a polynucleotide expressing a chimeric antigen receptor comprising a binding domain that specifically binds to B cell maturation antigen; and culturing the isolated marrow infiltrating lymphocytes in the presence of IL-2, IL-7, IL-15, and IL-21.

The chimeric antigen receptor, i.e. BCMA CAR, comprising a binding domain that specifically binds to B cell maturation antigen used in the above method may be one described in the "chimeric antigen receptor according to the present invention" section above.

Specifically, the BCMA CAR used in the method according to the present invention may have at least 70%, preferably at least 80%, and more preferably at least 90%, 95%, 97%, 99% or 100% sequence identity with the amino acid sequence of SEQ ID NO: 1.

The binding domain included in the BCMA CAR used in the method according to the present invention includes VHH01 and VHH02, which are nanobodies specific for BCMA, and the above nanobodies may have at least 70%, preferably at least 80%, and more preferably at least 90%, 95%, 97%, 99% or 100% sequence identity with the amino acid sequences of SEQ ID NOs: 8 and 9, respectively.

The binding domain included in the chimeric antigen receptor used in the method according to the present invention may include a VHH01 nanobody comprising the amino acid sequence of SEQ ID NO: 8 and a VHH02 nanobody comprising the amino acid sequence of SEQ ID NO: 9.

The VHH01 and VHH02 used in the method according to the present invention may comprise CDR1 of SEQ ID NO: 2 or 3, CDR2 of SEQ ID NO: 4 or 5, and CDR3 of SEQ ID NO: 6 or 7.

The VHH01 used in the method according to the present invention may comprise CDR1 of SEQ ID NO: 2, CDR2 of SEQ ID NO: 4, and CDR3 of SEQ ID NO: 6, and the VHH02 may comprise CDR1 of SEQ ID NO: 3, CDR2 of SEQ ID NO: 5, and CDR3 of SEQ ID NO: 7.

The BCMA CAR used in the method according to the present invention may further comprise one or more selected from the group consisting of a binding domain that specifically binds to BCMA, a signal sequence (signal peptide; SP), a hinge and transmembrane domain (TM), and an intracellular domain (intracellular signaling domain).

The hinge and transmembrane domain is preferably a hinge and transmembrane domain derived from CD8α, but is not limited thereto, and may preferably comprise the amino acid sequence of SEQ ID NO: 10, and may have at least 70%, preferably at least 80%, and more preferably at least 90%, 95%, 97% or 99% sequence identity with the amino acid sequence of SEQ ID NO: 10.

The intracellular domain of the BCMA CAR used in the method according to the present invention may include two or more intracellular signaling domains. The intracellular signaling domain is preferably an intracellular signaling domain derived from 4-1BB connected to an intracellular signaling domain derived from CD3ζ, but is not limited thereto. Preferably, the intracellular signaling domain derived from 4-1BB may comprise the amino acid sequence of SEQ ID NO: 11, and the intracellular signaling domain derived from CD3ζ may comprise the amino acid sequence of SEQ ID NO: 12, and may have at least 70%, preferably at least 80%, and more preferably at least 90%, 95%, 97% or 99% sequence identity with the amino acid sequence of SEQ ID NO: 11 or SEQ ID NO: 12.

The present invention also relates to eMIL produced by the above-described production method, that is, eMIL expressing BCMA CAR on the cell surface. As used herein, the fact that eMIL comprises BCMA CAR means that BCMA CAR is expressed on the cell surface of eMIL.

The population of CAR-MILs prepared by the method of the present invention may be a population that exhibits an increased proportion of CD8⁺ T cells and a reduced proportion of CD4⁺ T cells compared to the MIL population that is just isolated from a patient.

The CAR-MILs prepared by the method of the present invention may be a population that exhibits a reduced proportion of regulatory T cells compared to the MIL population that is just isolated from a patient.

The CAR-MILs prepared by the method of the present invention may be a population that exhibits an increased proportion of CD62L⁺ CD45RA⁻ central memory T cells compared to the MIL population that is just isolated from a patient.

The CAR-MILs prepared by the method of the present invention may be a population that exhibits a reduced expression level of PD-1 (programmed cell death protein 1) compared to the MIL population that is just isolated from a patient.

The CAR-MILs prepared by the method of the present invention may exhibit an increased proportion of CD8⁺ T cells and central memory T cells, a reduced proportion of CD4⁺ T cells and regulatory T cells, and a low expression level of PD-1.

The CAR-MILs prepared by the method of the present invention may exhibit excellent cytotoxic capacity against CD138⁺ primary multiple myeloma cells with a higher expression rate of CD107a compared to the isolated peripheral blood lymphocytes or the isolated population of marrow infiltrating lymphocytes that do not express a chimeric antigen receptor.

As used herein, the term "cancer" may be specifically one or more selected from the group consisting of brain tumor, cervical cancer, ovarian cancer, prostate cancer, lung cancer, bile duct cancer, kidney cancer, gastric cancer, liver cancer, retinoblastoma, choriocarcinoma, small intestine cancer, large intestine cancer and rectal cancer, non-small cell lung cancer, gastric adenocarcinoma, acute lymphocytic leukemia, acute myeloid leukemia, breast cancer, bone sarcoma, bladder cancer, anaplastic astrocytoma, and multiple myeloma, but is not limited thereto.

As used herein, the term "multiple myeloma (MM)" refers to a type of blood cancer that occurs when plasma cells in the bone marrow abnormally differentiate and proliferate.

As used herein, the term "prevention" refers to any action that suppresses or delays the onset of a disease by the administration of a composition, and the term "treatment" refers to any action that improves or beneficially changes the symptoms of a subject suspected of having a disease and a subject who has developed a disease by the administration of a composition.

The administration dose of the cell population of CAR-MILs according to the present invention can be appropriately selected by those of ordinary skill in the art depending on the subject's condition, body weight, disease, formulation, administration route and period.

The cell population of CAR-MILs according to the present invention may be administered through all common routes to reach the target tissue. For example, it may be administered intravenously, subcutaneously, or intraperitoneally, but is not limited thereto.

As used herein, the term "combined administration" refers to simultaneous or sequential administration of two or more active ingredients.

The cell population of CAR-MILs according to the present invention may be administered in combination with other anticancer agents appropriately selected by those of ordinary skill in the art. For example, it may be administered in combination with an immune checkpoint inhibitor, but is not limited thereto.

Hereinafter, the present invention will be described in more detail through the examples, but the following examples are for illustrative purposes only and are not intended to limit the scope of the present invention.

### [Preparation Example 1]

### Production of CAR-MILs

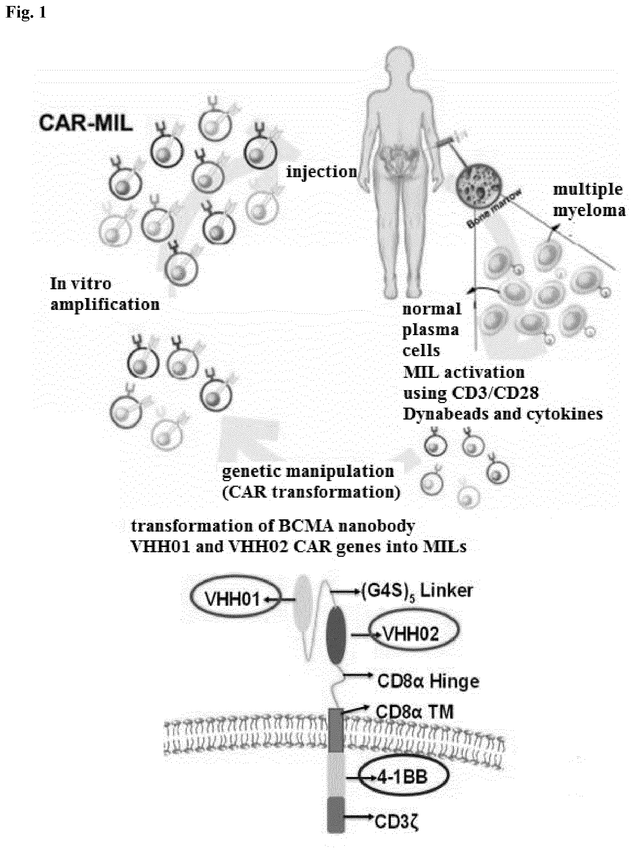

CAR-MILs were produced in the same manner as shown in Fig. 1. Specifically, bone marrow mononuclear cells ("BMMNC") were isolated from the bone marrow of patients with multiple myeloma using density gradient centrifugation using Ficoll-Hypaque (d = 1.077, Lymphoprep^{™} Axis-Shield, Oslo, Norway). Thereafter, BMMNC were co-cultured for 2 days with anti-CD3/CD28 antibody-coated beads in a 24 well plate in RPMI 1640 medium containing 10% FBS, 100 U/mL penicillin, 100 µg/mL streptomycin, 4 mmol/L L-glutamine, and 200 U/mL recombinant human IL-2. Thereafter, the BCMA CAR having the nucleic acid sequence of SEQ ID NO: 13 in Table 4 below was transduced into BMMNC and cultured in the RPMI medium supplemented with 10 ng/mL IL-7, 10 U/mL IL-15, and 5 ng/mL IL-21. The medium was changed every 2-3 days, and CAR-MILs were cultured continuously for up to 21 days. The BCMA CAR comprises two nanobodies, referred to as VHH01 and VHH02, and the CDR sequences of the above nanobodies are shown in Table 2 below. The BCMA CAR comprises VHH01 comprising the amino acid sequence of SEQ ID NO: 8, VHH02 comprising the amino acid sequence of SEQ ID NO: 9, a (G4S)s linker, a hinge and transmembrane domain of CD8α comprising the amino acid sequence of SEQ ID NO: 10, an intracellular signaling domain of 4-1BB comprising the amino acid sequence of SEQ ID NO: 11, and an intracellular signaling domain of CD3ζ comprising the amino acid sequence of SEQ ID NO: 12, and the sequences are shown in Table 3 below.

**[Table 1]**

| N a m e | S E Q I D N O | Amino acid sequence |
|---|---|---|
| B C M A C A R | 1 | QVQLQESGGGSVQPGGSLRLSCKASGFTFSPSAMSWARQAPGKEIEWVSNI YPDGRTYYADSMKGRFTISRDNAENTVYLQMNSLKPEDTAVYYCLIGVGI VAGQVVSPYWGQGTQVTVSSGGGGSGGGGSGGGGSGGGGSGGGGSQVQ LQESGGGLVQPGGSLRLSCAASGFTFSNYAMSWFRQGPGKERELVAGIDRT GGSTRTADSVQGRFTISRDNAKNKVYLQMNSLKPEDTAVYYCKALRTPG WGTSPDVVGYWGQGTQVTVSSTTPAPRPPTPAPTIASQPLSLRPEACRPAA GGAVHTRGLDFACDIYIWAPLAGTCGVLLLSLVITLYCKRGRKKLLYIFKQ PFMRPVQTTQEEDGCSCRFPEEEEGGCELRVKFSRSADAPAYKQGQNQLY NELNLGRREEYDVLDKRRGRDPEMGGKPRRKNPQEGLYNELQKDKMAEA YSEIGMKGERRRGKGHDGLYQGLSTATKDTYDALHMQALPPR |

**[Table 2]**

| Name of nanobody | CDR1 | CDR2 | CDR3 |
|---|---|---|---|
| VHH01 | GFTFSPSA | IYPDGRT | LIGVGIVAGQVVSPY |
| VHH02 | GFTFSNYA | IDRTGGST | KALRTPGWGTSPDVVGY |

**[Table 3]**

| Nam e | S E Q I D N O | Amino acid sequence |
|---|---|---|
| VHH 01 | 8 | QVQLQESGGGSVQPGGSLRLSCKASGFTFSPSAMSWARQAPGKEIEWV SNIYPDGRTYYADSMKGRFTISRDNAENTVYLQMNSLKPEDTAVYYC LIGVGIVAGQVVSPYWGQGTQVTVSS |
| VHH 02 | 9 | QVQLQESGGGLVQPGGSLRLSCAASGFTFSNYAMSWFRQGPGKEREL VAGIDRTGGSTRTADSVQGRFTISRDNAKNKVYLQMNSLKPEDTAVY YCKALRTPGWGTSPDVVGYWGQGTQVTVSS |
| hinge and trans mem brane doma in of CD8 α | 1 0 | TTPAPRPPTPAPTIASQPLSLRPEACRPAAGGAVHTRGLDFACDIYIWAP LAGTCGVLLLSLVITLYCKR |
| intrac ellula r signa ling doma in of 4-1BB | 1 1 | GRKKLLYIFKQPFMRPVQTTQEEDGCSCRFPEEEEGGCELRV |
| intrac ellula r signa ling | 1 2 | KFSRSADAPAYKQGQNQLYNELNLGRREEYDVLDKRRGRDPEMGGK PRRKNPQEGLYNELQKDKMAEAYSEIGMKGERRRGKGHDGLYQGLS TATKDTYDALHMQALPPR |
| doma in of CD3ζ | | |

**[Table 4]**

| N a m e | S E Q I D N O | Nucleic acid sequence |
|---|---|---|
| B C M A C A R | 1 3 | GGCTCCGGTGCCCGTCAGTGGGCAGAGCGCACATCGCCCACAGTCCCCG AGAAGTTGGGGGGAGGGGTCGGCAATTGAACCGGTGCCTAGAGAAGGT GGCGCGGGGTAAACTGGGAAAGTGATGTCGTGTACTGGCTCCGCCTTTT TCCCGAGGGTGGGGGAGAACCGTATATAAGTGCAGTAGTCGCCGTGAA CGTTCTTTTTCGCAACGGGTTTGCCGCCAGAACACAGGTAAGTGCCGTG TGTGGTTCCCGCGGGCCTGGCCTCTTTACGGGTTATGGCCCTTGCGTGCC TTGAATTACTTCCACCTGGCTGCAGTACGTGATTCTTGATCCCGAGCTTC GGGTTGGAAGTGGGTGGGAGAGTTCGAGGCCTTGCGCTTAAGGAGCCC CTTCGCCTCGTGCTTGAGTTGAGGCCTGGCCTGGGCGCTGGGGCCGCCG CGTGCGAATCTGGTGGCACCTTCGCGCCTGTCTCGCTGCTTTCGATAAGT CTCTAGCCATTTAAAATTTTTGATGACCTGCTGCGACGCTTTTTTTCTGG CAAGATAGTCTTGTAAATGCGGGCCAAGATCTGCACACTGGTATTTCGG TTTTTGGGGCCGCGGGCGGCGACGGGGCCCGTGCGTCCCAGCGCACATG TTCGGCGAGGCGGGGCCTGCGAGCGCGGCCACCGAGAATCGGACGGGG GTAGTCTCAAGCTGGCCGGCCTGCTCTGGTGCCTGGCCTCGCGCCGCCG TGTATCGCCCCGCCCTGGGCGGCAAGGCTGGCCCGGTCGGCACCAGTTG CGTGAGCGGAAAGATGGCCGCTTCCCGGCCCTGCTGCAGGGAGCTCAA AATGGAGGACGCGGCGCTCGGGAGAGCGGGCGGGTGAGTCACCCACAC AAAGGAAAAGGGCCTTTCCGTCCTCAGCCGTCGCTTCATGTGACTCCAC GGAGTACCGGGCGCCGTCCAGGCACCTCGATTAGTTCTCGAGCTTTTGG AGTACGTCGTCTTTAGGTTGGGGGGAGGGGTTTTATGCGATGGAGTTTC CCCACACTGAGTGGGTGGAGACTGAAGTTAGGCCAGCTTGGCACTTGAT GTAATTCTCCTTGGAATTTGCCCTTTTTGAGTTTGGATCTTGGTTCATTCT CAAGCCTCAGACAGTGGTTCAAAGTTTTTTTCTTCCATTTCAGGTGTCGT GAATGCTGCTGCTGGTGACCAGCCTGCTGCTGTGCGAGCTGCCCCACCC CGCCTTTCTGCTGATCCCCCAGGTGCAGCTGCAGGAGTCTGGAGGAGGC |
| | | TCGGTACAGCCTGGGGGGTCTCTGAGACTCTCCTGTAAAGCCTCTGGAT TCACCTTCAGTCCCTCTGCCATGAGCTGGGCCCGCCAGGCACCAGGGAA GGAGATCGAGTGGGTGTCCAATATTTACCCTGATGGACGCACATACTAT GCGGACTCCATGAAGGGCCGATTCACCATCTCCAGAGACAACGCCGAG AACACGGTGTATCTGCAAATGAACAGCCTGAAACCTGAGGACACGGCC GTTTATTACTGTCTAATAGGAGTCGGTATTGTGGCCGGTCAGGTAGTTTC GCCCTACTGGGGTCAGGGGACCCAGGTCACCGTCTCCTCAGGAGGCGG AGGATCTGGCGGAGGTGGAAGTGGCGGAGGCGGTTCTGGTGGTGGTGG CTCTGGTGGCGGCGGATCTCAGGTGCAGCTGCAGGAGTCTGGAGGAGG CTTGGTGCAGCCTGGGGGGTCTCTGAGACTCTCCTGTGCAGCCTCTGGA TTCACCTTCAGTAACTATGCCATGAGCTGGTTCCGCCAGGGTCCAGGGA AGGAGCGCGAGTTGGTCGCAGGTATCGATCGTACTGGAGGTAGCACAA GGACTGCAGACTCCGTGCAGGGCCGATTCACCATCTCCAGAGACAATGC CAAGAACAAGGTGTATCTGCAAATGAATAGCCTGAAACCTGAGGACAC GGCCGTGTATTATTGTAAGGCACTGAGGACTCCGGGCTGGGGTACGTCT CCCGATGTCGTTGGCTACTGGGGCCAGGGGACCCAGGTCACCGTCTCCT CAACTACCCCAGCACCGCGGCCGCCCACCCCGGCTCCTACCATCGCCTC CCAGCCTCTGTCCCTGCGTCCGGAGGCATGTAGACCCGCAGCTGGTGGG GCCGTGCATACCCGGGGTCTTGACTTCGCCTGCGATATCTACATTTGGG CCCCTCTGGCTGGTACTTGCGGGGTCCTGCTGCTTTCACTCGTGATCACT CTTTACTGTAAGCGCGGTCGGAAGAAGCTGCTGTACATCTTTAAGCAAC CCTTCATGAGGCCTGTGCAGACTACTCAAGAGGAGGACGGCTGTTCATG CCGGTTCCCAGAGGAGGAGGAAGGCGGCTGCGAACTGCGCGTGAAATT CAGCCGCAGCGCAGATGCTCCAGCCTACAAGCAGGGGCAGAACCAGCT CTACAACGAACTCAATCTTGGTCGGAGAGAGGAGTACGACGTGCTGGA CAAGCGGAGAGGACGGGACCCAGAAATGGGCGGGAAGCCGCGCAGAA AGAATCCCCAAGAGGGCCTGTACAACGAGCTCCAAAAGGATAAGATGG CAGAAGCCTATAGCGAGATTGGTATGAAAGGGGAACGCAGAAGAGGCA AAGGCCACGACGGACTGTACCAGGGACTCAGCACCGCCACCAAGGACA CCTATGACGCTCTTCACATGCAGGCCCTGCCGCCTCGG |

### [Example 1]

### Analysis of immune population of CAR-MILs

In order to analyze the immune cell population of CAR-MILs, the CAR-MILs produced by the method of Preparation Example 1 above were treated with a fluorescently labeled monoclonal antibody, and samples were collected using a flow cytometer, and then the expression of cell surface markers was analyzed to confirm the composition of the immune cell population.

Specifically, in order to analyze CD3⁺ cells, NK cells (natural killer cells), CIK cells (cytokine-induced killer cells), regulatory T cells (T_{reg}), CD4⁺ T cells, and CD8⁺ T cells, etc., the composition of the immune cell population was confirmed using surface monoclonal antibodies such as CD3-FITC, CD56-PE-Cy7, CD4-PE, CD8-Amyan, CD62L-PE-Cy7, CD45RA, PD-1-Pacific blue, TIGIT-APC, TIM3-PerCP-Cy5.5, and CD73-APC-Cy7.

First, in order to confirm the transduction efficiency of the CAR according to the present invention in BCMA CAR-MILs, CAR-MILs (2 × 10⁵ cells) were washed with FACS buffer, then stained with the biotinylated human BCMA and Live/Dead-AmCyan (Invitrogen, Carlsbad, CA), and treated with Fc blocker. Thereafter, the cells were washed twice and then incubated with PE streptavidin antibody at room temperature for 30 minutes. Thereafter, the prepared samples were analyzed using a flow cytometer (BD FACS Canto II, Becton Dickinson, Mountain View, CA, USA) to collect data, and the expression of the cell surface markers was analyzed using Flow Jo software (TreeStar, San Carlos, CA, USA) to confirm the transduction efficiency of BCMA CAR-MILs.

The composition of the immune cell population was also performed in the same manner as above, while changing the monoclonal antibody.

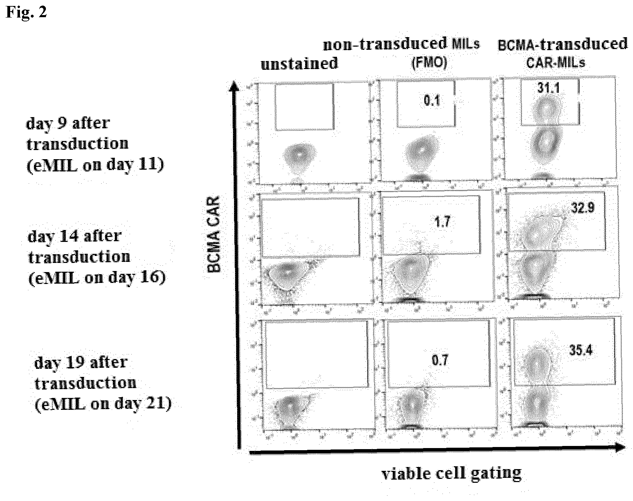

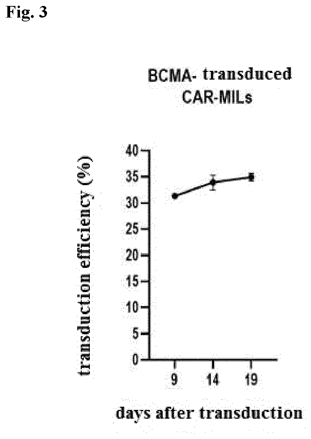

As a result, as shown in Figs. 2 and 3, the expression rate of CAR in CAR-MILs continued to increase for 21 days, and the expression rate of BCMA CAR was about 31.1% on day 9 of culture compared to day 0 of culture, and it was 35.4% on day 21, which showed an excellent expression rate due to the small size of the nanobody.

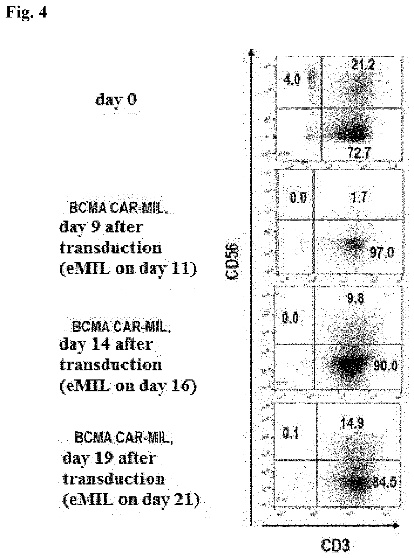

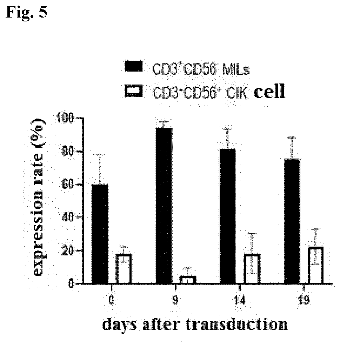

As shown in Figs. 4 and 5, the BCMA CAR-MILs of the present invention were confirmed to comprise a small number of cytokine-induced killer cells (CD3⁺CD56⁺).

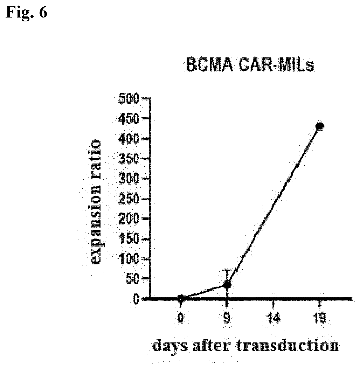

In addition, as shown in Fig. 6, the BCMA CAR-MILs of the present invention exhibit a proliferation rate approximately 400 times higher on day 19 after transduction in vitro, demonstrating that they can be efficiently used in cancer treatment due to this excellent proliferation rate.

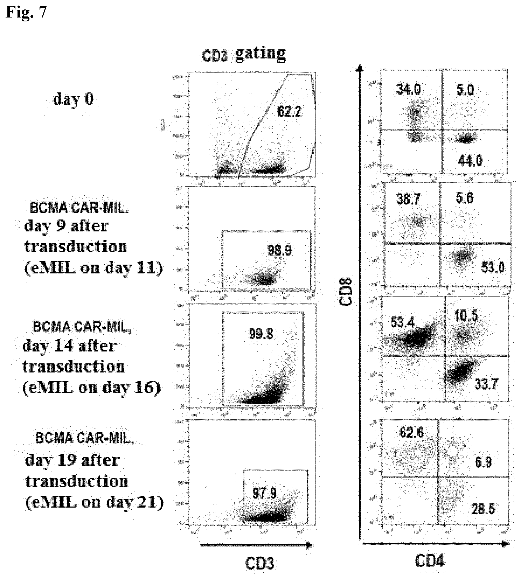

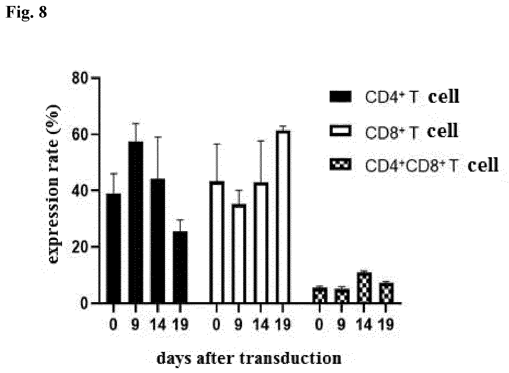

In addition, as shown in Figs. 7 and 8, it was confirmed that after transduction of the CAR according to the present invention, the proportion of CD8⁺ T cells continued to increase, and the proportion of CD4⁺ T cells was decreased, indicating that T cells showing cytotoxicity was increased.

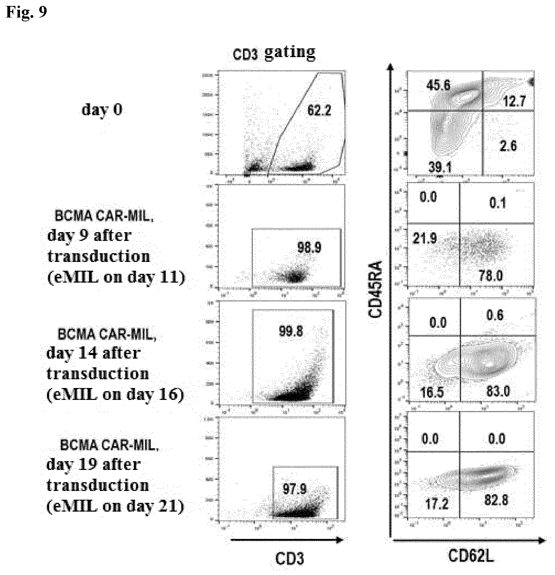

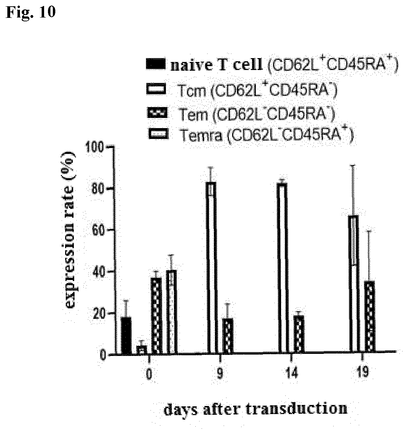

In addition, as shown in Figs. 9 and 10, it was confirmed that after transduction of the CAR according to the present invention, the proportion of central memory T cells (CD62L⁺CD45RA⁻), also referred to as T_{cm}, was increased significantly, and there were almost no populations of naive T cells and Tₑₘᵣₐ (CD62L⁻CD45RA⁺) cells.

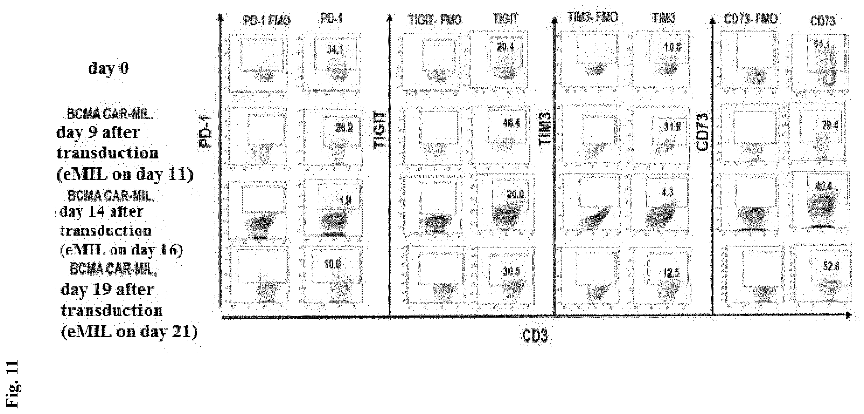

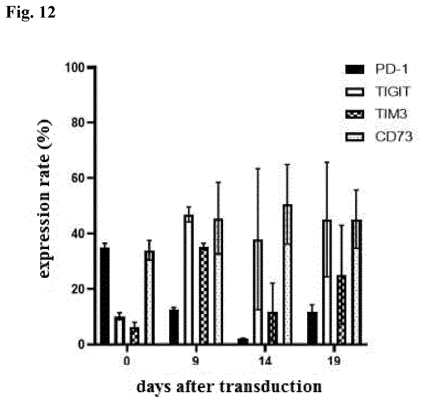

Additionally, as shown in Figs. 11 and 12, it was confirmed that the CAR-MILs of the present invention can overcome the immunosuppression or immune evasion mechanism of cancer cells by expressing immune checkpoint proteins such as PD-1 at a significantly low level.

### [Example 2]

### Analysis of Treg cell proportion in population of CAR-MILs

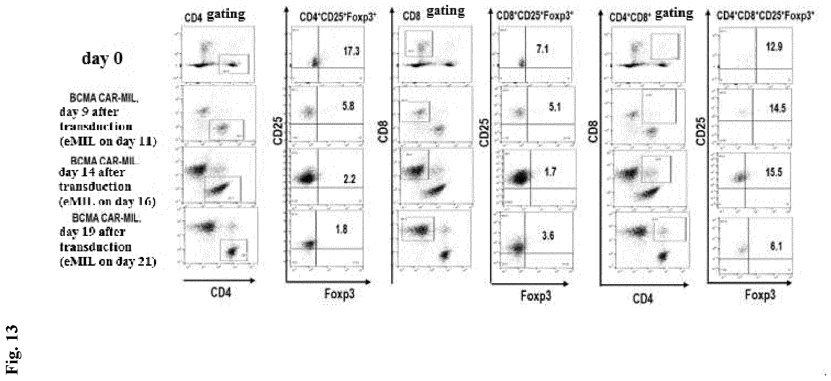

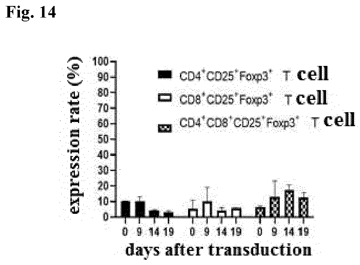

The composition of the immune cell population of CAR-MILs was analyzed in the same manner as in Example 1. As a result, as shown in Figs. 13 and 14, it was confirmed that the expression rate of CD4⁺CD25⁺Foxp3⁺ regulatory T cells (T_{reg}) and CD8⁺CD25⁺Foxp3⁺ regulatory T cells (T_{reg}) was decreased.

Therefore, it was confirmed that the immune cell population of CAR-MILs produced by the method of the present invention contained almost no immunosuppressive cells (T_{reg}).

### [Example 3]

### Cytotoxicity analysis of CAR-MILs

In order to confirm whether CAR-MILs effectively kill multiple myeloma cells, an experiment was performed on the functional differences between CAR-MILs and activated MIL (eMIL) and activated peripheral blood lymphocytes (ePBL). Specifically, in order to evaluate the killing ability of CAR-MILs, LDH (lactate dehydrogenase) analysis, kinetic analysis, and CD107a degranulation analysis were performed.

### 3-1. CD107a degranulation analysis of CAR-MILs

CD107a degranulation analysis was performed as follows. 5×10⁴ CAR-MILs were cultured in a 96 well U-bottom plate with or without 5×10⁴ target cells (K562, U266, RPMI8226, ARH77 and IM9 and CD138⁺ multiple myeloma cells isolated from patients). The cells were cultured with 5 µL of PE-conjugated anti-human CD107a antibody in a 96 well U-bottom plate with or without 5×10⁴ target cells (K562, U266, RPMI8226, ARH77, IM9 and CD138⁺ primary multiple myeloma cells). After 1 hour, monensin and brefeldin A (BD Biosciences) were added and cultured for an additional 4 hours. Thereafter, after staining with anti-human CD3 antibody, the cells were obtained.

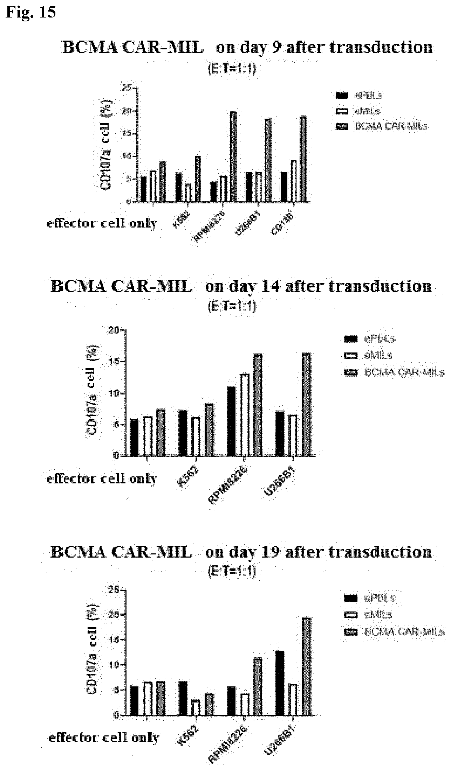

As shown in Fig. 15, it was confirmed that CAR-MILs obtained on day 9, CAR-MILs obtained on day 14, and CAR-MILs obtained on day 21 after transduction of the CAR according to the present invention exhibit a higher proportion of the CD107a⁺ positive population compared to ePBL and eMIL at an E (Effector cell; CAR-MILs) : T (Target cell; target cell) 1: 1 ratio, thereby confirming that they exhibit a higher cell killing activity against cancer cells.

### 3-2. LDH analysis of tumor specific cytotoxicity of CAR-MILs

LDH analysis was performed as follows. For target cells (K562, U266, RPMI8226, and CD138⁺ multiple myeloma cells), 10⁶ cells were added to 100 µL of RPMI medium with 2 µg of anti-human HLA-A, B or C antibody (clone: W6/32, Biolegend, USA), cultured for 20 minutes, and then washed by centrifugation. Thereafter, the target cells were co-cultured with CAR-MII, at a 1:1 ratio in a Costar 96-well plate (Corning, USA) at 5% CO₂, 37 °C for 6 hours. Finally, in order to measure the concentration of LDH, a cytosolic enzyme released during cell lysis, the supernatant was collected, and the concentration of LDH was measured using CytoTox 96 non-radioactive cytotoxicity assay (Promega, USA), and the cell lysis rate was calculated according to the manufacturer's protocol.

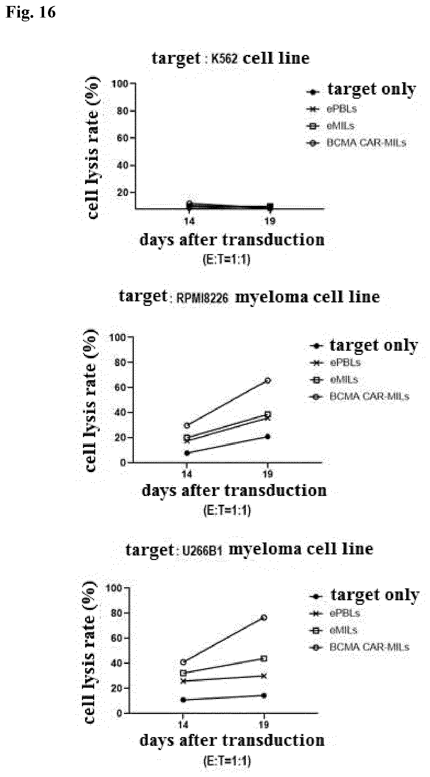

CAR-MILs, eMIL and ePBL were cultured with the target leukemia (K562) cells that do not express BCMA or the target myeloma cells (U266, RPMI8226, and CD138⁺ primary multiple myeloma cells) that express BCMA, and then the cell lysis rate was calculated. As a result, as shown in Fig. 16, it was confirmed that CAR-MILs showed no difference in cytotoxicity against K562 cells that do not express BCMA, which were used as a negative control, but showed significantly increased cytotoxicity against U266 and RPMI8226 that overexpress BCMA.

### 3-3. Kinetic analysis of tumor specific cytotoxicity of CAR-MILs

In order to evaluate the cytotoxicity of BCMA CAR-MILs against cancer cells in real time, IncuCyte^{®} Cytotoxicity Assay (Satorius) was performed. As target cells, RPMI8226 and CD138+ primary multiple myeloma cells isolated from patients with multiple myeloma were spread in a 96-well plate coated with poly-L-ornithine solution (Sigma-Aldrich) at 1 × 10⁴ cells (per 100 µL), and BCMA CAR-MILs were added in an effector cell:target cell (E:T) ratio of 1:1. IncuCyte^{®} Cytotox Green Reagent (Satorius) was added at a 1:1000 dilution (100 nL) to the mixed cells, and then the cell plate was placed in the Incucyte^{®} Live-Cell Analysis System (Satorius) and reacted at 37 °C for 30 minutes. The number of apoptotic cells identified by IncuCyte^{®} Cytotox Green Reagent was monitored using the IncuCyte Live Cell Analysis System, and fluorescent images were captured every 2 hours for 24 hours to confirm cytotoxicity.

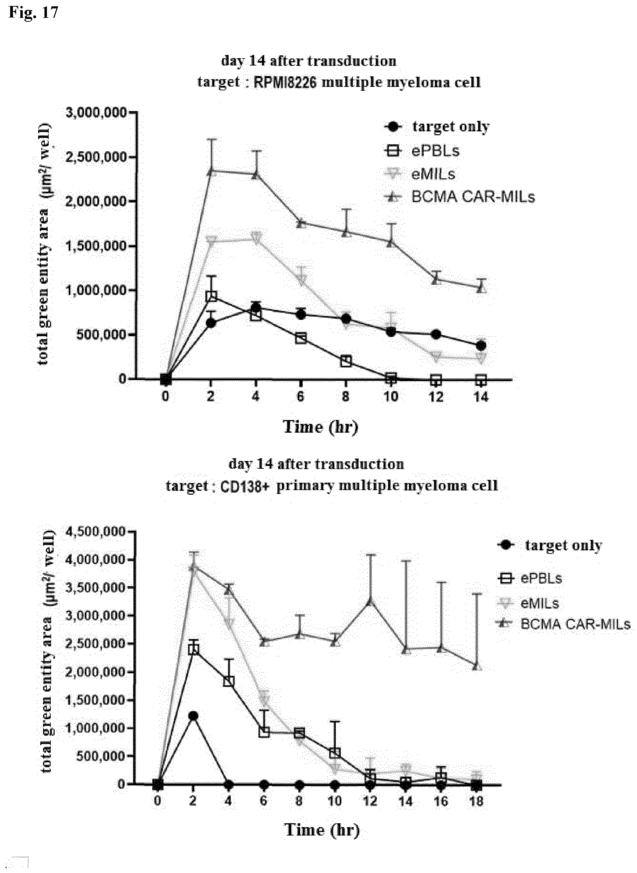

As shown in Fig. 17, it was confirmed that in the BCMA CAR-MII, treatment group, the number of green entities shown by IncuCyte^{®} Cytotox Green Reagent, which specifically binds to apoptotic cells, was increased significantly and was maintained continuously. Therefore, it was confirmed that the CAR-MILs of the present invention consistently exhibit higher cell killing activity compared to ePBL and eMIL.

Finally, as shown in Examples 3-1 to 3-3, it was confirmed that the CAR-MILs of the present invention exhibit excellent cytotoxicity against target cancer cells compared to MIL and PBL that do not express CAR. In addition, an experiment was performed on the functional differences between CAR-T and CAR-MIL, and it was confirmed that CAR-MII, exhibits excellent cytotoxicity against cancer cells.

### Aspects of the Invention

### [Aspect 1]

An isolated population of marrow infiltrating lymphocytes (MILs) comprising a chimeric antigen receptor (CAR) comprising a binding domain that specifically binds to B cell maturation antigen (BCMA).

### [Aspect 2]

The isolated population of marrow infiltrating lymphocytes according to aspect 1, wherein the isolated population of marrow infiltrating lymphocytes comprises a chimeric receptor further comprising one or more selected from the group consisting of a signal sequence, a linker, a hinge and transmembrane domain (TM), and an intracellular domain.

### [Aspect 3]

The isolated population of marrow infiltrating lymphocytes according to aspect 1, wherein the binding domain that specifically binds to B cell maturation antigen comprises a nanobody comprising CDR1 of SEQ ID NO: 2 or 3, CDR2 of SEQ ID NO: 4 or 5, and CDR3 of SEQ ID NO: 6 or 7.

### [Aspect 4]

The isolated population of marrow infiltrating lymphocytes according to aspect 1, wherein the binding domain that specifically binds to B cell maturation antigen comprises a nanobody comprising CDR1 of SEQ ID NO: 2, CDR2 of SEQ ID NO: 4, and CDR3 of SEQ ID NO: 6, and a nanobody comprising CDR1 of SEQ ID NO: 3, CDR2 of SEQ ID NO: 5, and CDR3 of SEQ ID NO: 7.

### [Aspect 5]

The isolated population of marrow infiltrating lymphocytes according to aspect 1, wherein the binding domain that specifically binds to B cell maturation antigen comprises a nanobody comprising an amino acid sequence selected from the group consisting of the amino acid sequences of SEQ ID NOs: 8 and 9.

### [Aspect 6]

The isolated population of marrow infiltrating lymphocytes according to aspect 1, wherein the binding domain that specifically binds to B cell maturation antigen comprises a nanobody comprising the amino acid sequence of SEQ ID NO: 8 and a nanobody comprising the amino acid sequence of SEQ ID NO: 9.

### [Aspect 7]

The isolated population of marrow infiltrating lymphocytes according to aspect 1, wherein the chimeric antigen receptor comprises the amino acid sequence of SEQ ID NO: 1.

### [Aspect 8]

The isolated population of marrow infiltrating lymphocytes according to aspect 2, wherein the hinge and transmembrane domain comprises a hinge and transmembrane domain of CD8α.

### [Aspect 9]

The isolated population of marrow infiltrating lymphocytes according to aspect 8, wherein the hinge and transmembrane domain of CD8α comprises the amino acid sequence of SEQ ID NO: 10.

### [Aspect 10]

The isolated population of marrow infiltrating lymphocytes according to aspect 2, wherein the intracellular domain comprises a sequence selected from the group consisting of an intracellular signal region sequence selected from the group consisting of 4-1BB and CD3ζ, or a combination thereof.

### [Aspect 11]

The isolated population of marrow infiltrating lymphocytes according to aspect 10, wherein the intracellular signal region sequence of 4-1BB comprises the amino acid sequence of SEQ ID NO: 11, and the intracellular signal region sequence of CD3ζ comprises the amino acid sequence of SEQ ID NO: 12.

### [Aspect 12]

The isolated population of marrow infiltrating lymphocytes according to aspect 1, wherein the isolated population of marrow infiltrating lymphocytes exhibits an increased proportion of CD8⁺ T cells and a reduced proportion of CD4⁺ T cells compared to an isolated population of marrow infiltrating lymphocytes that is not activated in vitro and does not comprise a chimeric antigen receptor that specifically binds to B cell maturation antigen.

### [Aspect 13]

The isolated population of marrow infiltrating lymphocytes according to aspect 1, wherein the isolated population of marrow infiltrating lymphocytes exhibits a reduced proportion of regulatory T cells compared to an isolated population of marrow infiltrating lymphocytes that is not activated in vitro and does not comprise a chimeric antigen receptor that specifically binds to B cell maturation antigen.

### [Aspect 14]

The isolated population of marrow infiltrating lymphocytes according to aspect 1, wherein the isolated population of marrow infiltrating lymphocytes exhibits an increased proportion of central memory T cells compared to an isolated population of marrow infiltrating lymphocytes that is not activated in vitro and does not comprise a chimeric antigen receptor that specifically binds to B cell maturation antigen.

### [Aspect 15]

The isolated population of marrow infiltrating lymphocytes according to aspect 1, wherein the isolated population of marrow infiltrating lymphocytes exhibits a reduced expression level of PD-1 (programmed cell death protein 1) compared to an isolated population of marrow infiltrating lymphocytes that is not activated in vitro and does not comprise a chimeric antigen receptor that specifically binds to B cell maturation antigen.

### [Aspect 16]

The isolated population of marrow infiltrating lymphocytes according to aspect 1, wherein the isolated population of marrow infiltrating lymphocytes exhibits an increased proportion of CD8⁺ T cells and central memory T cells, a reduced proportion of CD4⁺ T cells and regulatory T cells, and a low expression level of PD-1 compared to an isolated population of marrow infiltrating lymphocytes that is not activated in vitro and does not comprise a chimeric antigen receptor that specifically binds to B cell maturation antigen.

### [Aspect 17]

The isolated population of marrow infiltrating lymphocytes according to aspect 1, wherein the isolated population of marrow infiltrating lymphocytes exhibits excellent cytotoxic capacity against CD138⁺ primary multiple myeloma cells with a higher expression rate of CD107a compared to an isolated population of peripheral blood lymphocytes or marrow infiltrating lymphocytes that is not activated in vitro and does not comprise a chimeric antigen receptor that specifically binds to B cell maturation antigen.

### [Aspect 18]

The isolated population of marrow infiltrating lymphocytes according to aspect 1, wherein the isolated marrow infiltrating lymphocytes are obtained by a method comprising the steps of:
(a) isolating marrow infiltrating lymphocytes (MILs) from the bone marrow of a patient with cancer;
(b) culturing the marrow infiltrating lymphocytes obtained from step (a) by contacting them with anti-CD3 and anti-CD28 antibodies in the presence of IL-2;
(c) transducing the marrow infiltrating lymphocytes obtained from step (b) with a polynucleotide expressing a chimeric antigen receptor comprising a binding domain that specifically binds to B cell maturation antigen; and
(d) culturing the marrow infiltrating lymphocytes obtained from step (c) in the presence of IL-2, IL-7, IL-15, and IL-21.

### [Aspect 19]

The isolated population of marrow infiltrating lymphocytes according to aspect 18, wherein the cancer is multiple myeloma.

### [Aspect 20]

A pharmaceutical composition comprising the isolated population of marrow infiltrating lymphocytes according to any one of aspects 1 to 19.

### [Aspect 21]

The pharmaceutical composition according to aspect 20, wherein the composition is for use in the treatment of a subject with cancer.

### [Aspect 22]

The pharmaceutical composition according to aspect 21, wherein the cancer is multiple myeloma.

### [Aspect 23]

A method for treating a subject with multiple myeloma, comprising administering the isolated population of marrow infiltrating lymphocytes according to any one of aspects 1 to 19 to a subject in need of treatment for cancer.

### [Aspect 24]

The method according to aspect 23, wherein the cancer is multiple myeloma.

## Claims

1. An isolated population of marrow infiltrating lymphocytes (MILs) comprising a chimeric antigen receptor (CAR) comprising a binding domain that specifically binds to B cell maturation antigen (BCMA).

2. The isolated population of marrow infiltrating lymphocytes according to claim 1, wherein the isolated population of marrow infiltrating lymphocytes comprises a chimeric receptor further comprising one or more selected from the group consisting of a signal sequence, a linker, a hinge and transmembrane domain (TM), and an intracellular domain.

3. The isolated population of marrow infiltrating lymphocytes according to claim 1, wherein the binding domain that specifically binds to B cell maturation antigen comprises a nanobody comprising CDR1 of SEQ ID NO: 2 or 3, CDR2 of SEQ ID NO: 4 or 5, and CDR3 of SEQ ID NO: 6 or 7.

4. The isolated population of marrow infiltrating lymphocytes according to claim 1, wherein the binding domain that specifically binds to B cell maturation antigen comprises a nanobody comprising CDR1 of SEQ ID NO: 2, CDR2 of SEQ ID NO: 4, and CDR3 of SEQ ID NO: 6, and a nanobody comprising CDR1 of SEQ ID NO: 3, CDR2 of SEQ ID NO: 5, and CDR3 of SEQ ID NO: 7.

5. The isolated population of marrow infiltrating lymphocytes according to claim 1, wherein the binding domain that specifically binds to B cell maturation antigen comprises a nanobody comprising an amino acid sequence:
i) selected from the group consisting of the amino acid sequences of SEQ ID NOs: 8 and 9; or
ii) of SEQ ID NO: 8 and a nanobody comprising the amino acid sequence of SEQ ID NO: 9.

6. The isolated population of marrow infiltrating lymphocytes according to claim 1, wherein the chimeric antigen receptor comprises the amino acid sequence of SEQ ID NO: 1.

7. The isolated population of marrow infiltrating lymphocytes according to claim 2, wherein the hinge and transmembrane domain comprises a hinge and transmembrane domain of CD8α, optionally
wherein the hinge and transmembrane domain of CD8α comprises the amino acid sequence of SEQ ID NO: 10.

8. The isolated population of marrow infiltrating lymphocytes according to claim 2, wherein the intracellular domain comprises a sequence selected from the group consisting of an intracellular signal region sequence selected from the group consisting of 4-1BB and CD3ζ, or a combination thereof, optionally.
wherein the intracellular signal region sequence of 4-1BB comprises the amino acid sequence of SEQ ID NO: 11, and the intracellular signal region sequence of CD3ζ comprises the amino acid sequence of SEQ ID NO: 12.

9. The isolated population of marrow infiltrating lymphocytes according to claim 1, wherein the isolated population of marrow infiltrating lymphocytes exhibits an increased proportion of CD8⁺ T cells and a reduced proportion of CD4⁺ T cells compared to an isolated population of marrow infiltrating lymphocytes that is not activated in vitro and does not comprise a chimeric antigen receptor that specifically binds to B cell maturation antigen.

10. The isolated population of marrow infiltrating lymphocytes according to claim 1, wherein the isolated population of marrow infiltrating lymphocytes exhibits:
i) a reduced proportion of regulatory T cells compared to an isolated population of marrow infiltrating lymphocytes that is not activated in vitro and does not comprise a chimeric antigen receptor that specifically binds to B cell maturation antigen; or
ii) an increased proportion of central memory T cells compared to an isolated population of marrow infiltrating lymphocytes that is not activated in vitro and does not comprise a chimeric antigen receptor that specifically binds to B cell maturation antigen; or
iii) a reduced expression level of PD-1 (programmed cell death protein 1) compared to an isolated population of marrow infiltrating lymphocytes that is not activated in vitro and does not comprise a chimeric antigen receptor that specifically binds to B cell maturation antigen; or
iv) an increased proportion of CD8⁺ T cells and central memory T cells, a reduced proportion of CD4⁺ T cells and regulatory T cells, and a low expression level of PD-1 compared to an isolated population of marrow infiltrating lymphocytes that is not activated in vitro and does not comprise a chimeric antigen receptor that specifically binds to B cell maturation antigen.

11. The isolated population of marrow infiltrating lymphocytes according to claim 1, wherein the isolated population of marrow infiltrating lymphocytes exhibits excellent cytotoxic capacity against CD138⁺ primary multiple myeloma cells with a higher expression rate of CD107a compared to an isolated population of peripheral blood lymphocytes or marrow infiltrating lymphocytes that is not activated in vitro and does not comprise a chimeric antigen receptor that specifically binds to B cell maturation antigen.

12. The isolated population of marrow infiltrating lymphocytes according to claim 1, wherein the isolated marrow infiltrating lymphocytes are obtained by a method comprising the steps of:
(a) isolating marrow infiltrating lymphocytes (MILs) from the bone marrow of a patient with cancer;
(b) culturing the marrow infiltrating lymphocytes obtained from step (a) by contacting them with anti-CD3 and anti-CD28 antibodies in the presence of IL-2;
(c) transducing the marrow infiltrating lymphocytes obtained from step (b) with a polynucleotide expressing a chimeric antigen receptor comprising a binding domain that specifically binds to B cell maturation antigen; and
(d) culturing the marrow infiltrating lymphocytes obtained from step (c) in the presence of IL-2, IL-7, IL-15, and IL-21.

13. The isolated population of marrow infiltrating lymphocytes according to claim 12, wherein the cancer is multiple myeloma.

14. A pharmaceutical composition comprising the isolated population of marrow infiltrating lymphocytes according to any one of claims 1 to 13, optionally
wherein the composition is for use in the treatment of a subject with cancer, and further optionally
wherein the cancer is multiple myeloma.

15. A method for treating a subject with multiple myeloma, comprising administering the isolated population of marrow infiltrating lymphocytes according to any one of claims 1 to 13 to a subject in need of treatment for cancer, optionally
wherein the cancer is multiple myeloma.
